# EUROPÄISCHE PATENTSCHRIFT

(11) **EP 0 707 565 B1**
(45) Veröffentlichungstag und Bekanntmachung des Hinweises auf die Patenterteilung: **14.10.1998**
(21) Anmeldenummer: 94924217.6
(22) Anmeldetag: 30.06.1994
(51) Int. Cl.: C07C 303/24, C07C 305/06, C11D 1/14

(54) **VERFAHREN ZUR HERSTELLUNG VON ALKYLSULFATEN**
METHOD OF PRODUCING ALKYL SULPHATES WITH IMPROVED PROPERTIES
PROCEDE DE FABRICATION D'ALKYLSULFATES A PROPRIETES AMELIOREES

(30) Priorität: 09.07.1993 DE 4322968
(43) Veröffentlichungstag der Anmeldung: 24.04.1996
(73) Patentinhaber: Henkel Kommanditgesellschaft auf Aktien, 40191 Düsseldorf (DE)
(72) Erfinder: BREUCKER, Christoph, D-42781 Haan (DE); KOMP, Horst, Dieter, D-40764 Langenfeld (DE); SALZ, Rainer, D-40589 Düsseldorf (DE); SCHAMBIL, Fred, D-40789 Monheim (DE); SCHMID, Karl, D-40822 Mettmann (DE)
(86) Internationale Anmeldenummer: EP9402145
(87) Internationale Veröffentlichungsnummer: WO9501959

(56) Entgegenhaltungen:
- EP-A- 0 401 642
- GB-A- 852 968
- GB-A- 863 179
- GB-A- 1 066 505
- US-A- 1 968 797

## Beschreibung

### Gebiet der Erfindung

Die Erfindung betrifft ein Verfahren zur Herstellung von Alkylsulfaten mit verbesserten Produkteigenschaften, bei dem man ausgewählte Fettalkoholgemische in an sich bekannter Weise sulfatiert und neutralisiert, die Verwendung der nach dem Verfahren erhältlichen Alkylsulfate zur Herstellung von Waschmitteln sowie die Verwendung der ausgewählten Fettalkoholgemische zur Herstellung von Alkylsulfaten.

### Stand der Technik

Alkylsulfate mit 16 bis 18 Kohlenstoffatomen, sogenannte "Talgalkoholsulfate", stellen anionische Tenside mit ausgezeichneten Detergenseigenschaften dar. Im Gegensatz beispielsweise zu den petrochemischen Alkylbenzolsulfonaten, basieren Alkylsulfate auf tierischen oder pflanzlichen Rohstoffen - beispielsweise Talg- oder Palmstearylalkohol - und genügen somit dem wachsenden Marktbedürfnis nach möglichst naturnahen Verbrauchsprodukten.

Obschon Alkylsulfate schon seit mehr als 50 Jahren bekannt sind und zu den ersten synthetischen Tensiden überhaupt zählen, finden sie erst jetzt allmählich Eingang in Markenprodukte. Mit ihrer großtechnischen Herstellung und Vermarktung werden jedoch auch zahlreiche Probleme bei ihrer Herstellung und Konfektionierung sichtbar, die Gegenstand einer Vielzahl von Publikationen und Patentanmeldungen sind. Zu den wichtigsten Problemen zählen dabei die Erniedrigung der Viskosität wäßriger Pasten und die Sicherstellung einer akzeptablen Farbqualität.

In der Deutschen Patentschrift **DE-C3 25 01 622** (Albright & Wilson) wird beispielsweise berichtet, daß man hochkonzentriert flüssige Alkylsulfatlösungen ohne Durchlaufen einer für Aniontenside typischen Gelphase erhalten kann, indem man die Neutralisation innerhalb eines bestimmten pH- und Konzentrationsbereiches durchführt. In den Deutschen Offenlegungsschriften **DE-A1 40 32 909** und **DE-A1 40 32 910** (Henkel) wird vorgeschlagen, Alkylsulfatpasten zur Senkung der Viskosität sulfonierte Triglyceride beizugeben bzw. die Sulfatierung der Fettalkohole in Gegenwart von ungesättigten Triglyceriden durchzuführen. Aus der **DE-A1 40 38 476** (Henkel) sind feste Waschmittel enthaltend Alkylsulfate, Natriumsulfat und Alumosilicate bekannt, die nach Extrusion trotz eines Wassergehaltes von ca. 40 Gew.-% fest und rieselfähig sind. Zur Verbesserung der Fließfähigkeit von Alkylsulfatpasten beispielsweise auf Basis von Kokos- oder Talgalkohol wird in der **DE-A1 41 05 581** (Henkel) der Einsatz von ausgesuchten Polyglycolethern, Alkylpolyglucosiden und Mischethern als Viskositätsreglern empfohlen. Für den gleichen Zweck werden in der **EP-A1 0 436 296** (ICI) Polyglycole, Polyglycolester und Triglyceride und in der **EP-A2 0 504 986** (Shell) Fettalkoholethoxylate, Alkylethersulfate und Polyethylenglycolsulfate vorgeschlagen. Gemäß der Lehre der **DE-A1 41 09 250** (Henkel) lassen sich hochkonzentrierte Alkylsulfat-Pasten erhalten, indem man Gemische von gesättigten und ungesättigten Fettalkoholen sulfatiert.

Die Zusammenstellung, die freilich keinen Anspruch auf Vollständigkeit erhebt, macht deutlich, daß der Stand der Technik bereits eine Vielzahl von Verfahren enthält, mit deren Hilfe spezifische Probleme bei der Herstellung von Alkylsulfaten durch Optimierung der Reaktion oder Eingriff in die Prozeßführung mehr oder weniger zufriedenstellend gelöst werden können.

Bislang offen ist jedoch beispielsweise das Problem der mangelnden Wasserlöslichkeit, das insbesondere bei Waschmittelextrudaten, die mechanisch verfestigt wurden, besteht. Dementsprechend besteht ein Bedarf nach alkylsulfathaltigen festen Waschmitteln, die im Einspülgang der Waschmaschine oder bei der Hand- wäsche leicht und vollständig gelöst werden. Obschon weiterhin in der Waschmaschine eine starke Schaumentwicklung unerwünscht ist, sollen die Alkylsulfate bei Einsatz in Hand- waschmitteln sehr wohl einen stabilen Schaum entwickeln. Ein drittes ungelöstes Problem besteht schließlich darin, daß der Sulfiergrad marktüblicher Alkylsulfate typischerweise unter 95 % liegt. In diesem Zusammenhang besteht ein Bedarf an Alkylsulfaten mit vermindertem Anteil unsulfatierten Fettalkohols.

Die komplexe Aufgabe der Erfindung hat somit darin bestanden, ein Verfahren zur Herstellung von Alkylsulfaten zu entwikkeln, die sich durch eine verbesserte Wasserlöslichkeit, insbesondere auch nach Kompaktierung, ein optimales Schaumvermögen und einen verbesserten Sulfiergrad auszeichnen.

### Beschreibung der Erfindung

Gegenstand der Erfindung ist ein Verfahren zur Herstellung von Alkylsulfaten mit verbesserten Produkteigenschaften, bei dem man Fettalkohole ("A") der Zusammensetzung
0 bis 1 Gew.-% C₁₀
4 bis 23 Gew.-% C₁₂
1 bis 13 Gew.-% C₁₄
19 bis 52 Gew.-% C₁₆
34 bis 62 Gew.-% C₁₈
0 bis 3 Gew.-% C₂₀₊

in an sich bekannter Weise mit gasförmigem Schwefeltrioxid oder Chlorsulfonsäure sulfatiert und anschließend mit Basen neutralisiert.

Überraschenderweise wurde gefunden, daß sich Alkylsulfate mit dem geschilderten komplexen Eigenschaftsprofil erhalten lassen, wenn man zu ihrer Herstellung Fettalkohole einer sehr speziellen Zusammensetzung einsetzt.

### Fettalkoholgemische

Die im Sinne der Erfindung einzusetzenden Fettalkohole können durch Abmischung bekannter, pflanzlicher und/oder tierischer Alkohole hergestellt werden.

Hierzu kommen beispielweise Gemische aus 70 bis 90, vorzugsweise 70 bis 75 Gew.-% eines Fettalkohols ("B") der Zusammensetzung
0 bis 2 Gew.-% C₁₂
1 bis 7 Gew.-% C₁₄
25 bis 35 Gew.-% C₁₆
60 bis 67 Gew.-% C₁₈
0 bis 3 Gew.-% C₂₀
0 bis 1 Gew.-% C₂₂
und 10 bis 30, vorzugsweise 25 bis 30 Gew.-% eines Fettalkohols ("C") der Zusammensetzung
0 bis 3 Gew.-% C₁₀
48 bis 58 Gew.-% C₁₂
19 bis 24 Gew.-% C₁₄
9 bis 12 Gew.-% C₁₆
11 bis 15 Gew.-% C₁₈
0 bis 1 Gew.-% C₂₀
in Betracht.

Ein anderes Beispiel zur Herstellung der erfindungsgemäß einzusetzenden Fettalkohole stellen Gemische aus 70 bis 90, vorzugsweise 70 bis 75 Gew.-% eines Fettalkohols ("D") der Zusammensetzung
0 bis 3 Gew.-% C₁₄
45 bis 55 Gew.-% C₁₆
45 bis 55 Gew.-% C₁₈
0 bis 3 Gew.-% C₂₀
und 10 bis 30, vorzugsweise 25 bis 30 Gew.-% eines Fettalkohols ("C") der Zusammensetzung
0 bis 3 Gew.-% C₁₀
48 bis 58 Gew.-% C₁₂
19 bis 24 Gew.-% C₁₄
9 bis 12 Gew.-% C₁₆
11 bis 15 Gew.-% C₁₈
0 bis 1 Gew.-% C₂₀
dar.

Ein weiteres Beispiel zur Herstellung der erfindungsgemäß einzusetzenden Fettalkohole stellen Gemische aus 70 bis 95, vorzugsweise 85 bis 90 Gew.-% eines Fettalkohols ("B") der Zusammensetzung
0 bis 2 Gew.-% C₁₂
1 bis 7 Gew.-% C₁₄
25 bis 35 Gew.-% C₁₆
60 bis 67 Gew.-% C₁₈
0 bis 3 Gew.-% C₂₀
0 bis 1 Gew.-% C₂₂
und 5 bis 30, vorzugsweise 10 bis 15 Gew.-% eines Fettalkohols ("E") der Zusammensetzung
0 bis 2 Gew.-% C₁₀
65 bis 75 Gew.-% C₁₂
22 bis 30 Gew.-% C₁₄
0 bis 8 Gew.-% C₁₆
0 bis 1 Gew.-% C₁₈
dar.

Ein viertes Beispiel zur Herstellung der erfindungsgemäß einzusetzenden Fettalkohole stellen schließlich Gemische aus 70 bis 95, vorzugsweise 85 bis 90 Gew.-% eines Fettalkohols ("D") der Zusammensetzung
0 bis 3 Gew.-% C₁₄
45 bis 55 Gew.-% C₁₆
45 bis 55 Gew.-% C₁₈
0 bis 3 Gew.-% C₂₀
und 5 bis 30, vorzugsweise 10 bis 15 Gew.-% eines Fettalkohols ("E") der Zusammensetzung
0 bis 2 Gew.-% C₁₀
65 bis 75 Gew.-% C₁₂
22 bis 30 Gew.-% C₁₄
0 bis 8 Gew.-% C₁₆
0 bis 1 Gew.-% C₁₈
dar. Bei dem Produkt "B" handelt es sich beispielsweise um ein Fettalkoholgemisch auf Basis tierischem Talg, einer 1:1-Mischung aus Palmstearin und Rapsöl bzw. einer 1:1-Mischung aus Palmstearin und des C_{16/18}-Anteils aus Kokos- bzw. Palmkernöl, bei den Produkten "C" und "E" um Fettalkoholgemische auf Basis Kokosöl, bei Produkt "D" um ein Fettalkoholgemisch auf Basis Palmstearin.

In einer bevorzugten Ausführungsform der Erfindung werden die Fettalkoholgemische zusammen mit 0,1 bis 10, vorzugsweise 0,5 bis 3 Gew.-% - bezogen auf Fettalkohol - Polyethylenglycol (durchschnittliches Molekulargewicht 2000 bis 20.000) sulfatiert. Diese Maßnahme führt zu einer Verbesserung der Lagerstabilität der resultierenden Alkylsulfate.

### Sulfatierung und Neutralisation

Die Sulfatierung der Fettalkoholgemische kann in der für Fettsäureniedrigalkylester bekannten Weise **[J.Falbe (ed.), "Surfactants in consumer products", Springer Verlag, Berlin-Heidelberg, 1987, S. 61]** erfolgen, wobei Reaktoren, die nach dem Fallfilmprinzip arbeiten, bevorzugt sind. Als Sulfiermittel kommen Chlorsulfonsäure und insbesondere gasförmiges Schwefeltrioxid in Betracht. Letzteres wird üblicherweise mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Das molare Einsatzverhältnis von Fettalkohol zu Sulfatierungsmittel kan 1 : 0,9 bis 1 : 1,2 und vorzugsweise 1 : 0,95 bis 1 : 1,1 betragen. Üblicherweise wird die Sulfatierung bei Temperaturen von 30 bis 70 durchgeführt. Im Hinblick auf die Viskosität der Einsatzstoffe einerseits und die Farbqualität der resultierenden Sulfatierungsprodukte andererseits, hat es sich als optimal erwiesen, die Reaktion in einem Temperaturbereich von 40 bis 60°C durchzuführen.

Die bei der Sulfatierung anfallenden sauren Sulfierprodukte werden in wäßrige Basen eingerührt, neutralisiert und auf einen pH- Wert von 6.5 bis 8.5 eingestellt. Als Basen für die **Neutralisation** kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine sowie Glucamine in Betracht. Die Neutralisationsbasen gelangen dabei vorzugsweise in Form 5 bis 55 gew.- %iger wäßriger Lösungen zum Einsatz, wobei 5 bis 25 gew.-%ige wäßrige Natriumhydroxidlösungen bevorzugt sind. Gleichfalls geeignet sind in diesem Zusammenhang Verfahren zur Trocken- bzw. Sprühneutralisation.

Die Sulfatierungsprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfatierungsprodukte, 0,2 bis 2 Gew.-% Wasserstoffperoxid, berechnet als 100 %ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die nach dem erfindungsgemäßen Verfahren erhältlichen Alkylsulfate weisen einen hohen Sulfiergrad auf, sind leicht wasserlöslich, zeigen ein insbesondere für die Handwäsche optimales Schaumvermögen und sind auch in kaltem Wasser leicht löslich. Diese vorteilhaften Eigenschaften lassen sich auch auf die auf Basis von Alkylsulfaten durch Verfestigung herstellbaren Waschmittel übertragen.

Ein weiterer Gegenstand der Erfindung betrifft daher die Verwendung dieser Alkylsulfate zur Herstellung von Waschmitteln, insbesondere solchen, die ihrerseits nach dem Extrusionsverfahren hergestellt werden.

Ein weiterer Gegenstand der Erfindung betrifft schließlich die Verwendung von Fettalkoholen ("A") der Zusammensetzung
0 bis 1 Gew.-% C₁₀
4 bis 23 Gew.-% C₁₂
1 bis 13 Gew.-% C₁₄
19 bis 52 Gew.-% C₁₆
34 bis 62 Gew.-% C₁₈
0 bis 3 Gew.-% C₂₀₊
zur Herstellung von Alkylsulfaten mit verbesserten Produkteigenschaften.

Die folgenden Beispiele sollen den Gegenstand der Erfindung näher erläutern, ohne ihn darauf einzuschränken.

### Beispiele

### I. Eingesetzte Fettalkoholgemische

**Tab.1:**

| Zusammensetzung der Fettalkoholgemische | | | | | | |
|---|---|---|---|---|---|---|
| FA | Prod.A Gew.-% | Prod.B Gew.-% | Prod.C Gew.-% | Prod.D Gew.-% | Prod.E Gew.-% | Prod.Z Gew.-% |
| C10 | 0- 1 | | 0- 3 | | 0- 2 | |
| C12 | 4-23 | 0- 2 | 48-58 | | 65-75 | |
| C14 | 1-13 | 1- 7 | 19-24 | 0- 3 | 22-30 | |
| C16 | 19-52 | 25-35 | 9-12 | 45-55 | 0- 8 | 50 |
| C18 | 34-62 | 60-67 | 11-15 | 45-55 | 0- 1 | 50 |
| C20 | 0- 3 | 0- 3 | 0- 1 | 0- 3 | | |
| C22 | | 0- 1 | | | | |
| Legende: FA : Kettenlänge des Fettalkohols | | | | | | |

### II. Herstellungsbeispiele

### Beispiel 1:

In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden 5,0 mol einer Fettalkoholmischung gemäß Produkt A mit einem Schmelzpunkt von 43°C bis 55°C mit 5,35 mol gasförmigem Schwefeltrioxid (Alkohol : SO₃ = 1 : 1,07) zur Reaktion gebracht. Das saure Reaktionsgemisch wurde kontinuierlich in 25 gew.-%ige wäßrige Natriumhydroxidlösung eingetragen, dabei neutralisiert und auf einen pH-Wert von 10,9 eingestellt. Der Sulfiergrad des resultierenden Alkylsulfats betrug 97 %.

### Kenndaten des Produktes:

- Aniontensidgehalt :: 64,4 Gew.-%
- Unsulfierte Anteile :: 1,8 Gew.-%
- Natriumsulfat :: 1,2 Gew.-%
- Wasser :: 32,6 Gew.-%
- Klettfarbe :: 42

Der Aniontensidgehalt (WAS) sowie die Unsulfierten Anteile (US) wurden nach den DGF-Einheitsmethoden, Stuttgart 1950-1984, H-III-10 und G-II-6b ermittelt. Die Bestimmung der Klett-Farbzahl erfolgte nach 30 minütiger Bleiche mit 1 Gew.-% einer 35 gew.-%igen wäßrigen Wasserstoffperoxidlösung. Die Messung wurde bei einer Konzentration von 5 Gew.-% Aniontensid, pH = 7 und unter Verwendung einer 5 cm-Rundküvette sowie eines Blaufilters (400 bis 465 nm) durchgeführt.

### Beispiel 2:

Beispiel 1 wurde unter Einsatz einer Mischung aus 30 Gew.-% technischem Kokosfettalkohol (Lorol^{(R)} T, Fa.Henkel KGaA, Düsseldorf/FRG = Prod.C) aus 70 Gew.-% eines Talgfettalkoholschnitts (Hydrenol^{(R)} DV, Fa.Henkel KGaA, Düsseldorf/ FRG = Prod.B) mit einem Schmelzpunkt von 43°C wiederholt. Der Sulfiergrad des resultierenden Alkylsulfats betrug 97 %.

### Beispiel 3:

Beispiel 1 wurde unter Einsatz einer Mischung aus 30 Gew.-% technischem Kokosfettalkohol (Lorol^{(R)} T, Fa.Henkel KGaA, Düsseldorf/FRG = Prod.C) und 70 Gew.-% Palmstearinalkohol (Produkt D) mit einem Schmelzpunkt von 43°C wiederholt. Der Sulfiergrad des resultierenden Alkylsulfats betrug 97 %.

### Beispiel 4:

Beispiel 1 wurde unter Einsatz einer Mischung aus 17 Gew.-% technischem Kokosfettalkohol (Lorol^{(R)} Spezial, Fa.Henkel KGaA, Düsseldorf/FRG = Prod.E) und 83 Gew.-% eines Talgfettalkoholschnitts (Hydrenol^{(R)} DV, Fa.Henkel KGaA, Düsseldorf/FRG = Prod.B) mit einem Schmelzpunkt von 43°C wiederholt. Der Sulfiergrad des resultierenden Alkylsulfats betrug 97 %.

### Vergleichsbeispiel H1:

Beispiel 1 wurde unter Einsatz eines technischen Talgfettalkohols (Prod.Z) mit einem Schmelzpunkt von 53°C wiederholt. Der Sulfiergrad des resultierenden Alkylsulfats betrug 94 %.

### III. Anwendungstechnische Untersuchungen

**Schaummessung**. In einem doppelwandigen 2-1-Meßzylinder werden 500 ml entionisiertes Wasser mit 1,2 g Alkylsulfat (berechnet als Aktivsubstanz) versetzt und auf 30°C temperiert. Mit Hilfe einer Schlauchpumpe wird die Flüssigkeit mit einer Umlaufgeschwindigkeit von 41/min umgepumpt. Dabei wird die Prüfflotte ca. 5 mm über dem Boden des Meßzylinders mittels eines Glasrohres, das über einen Schlauch mit einer Pumpe verbunden ist, angesaugt und über ein zweites Glasrohr, das an der 2000-ml-Marke des Meßzylinders angebracht ist, im freien Fall zurückgeführt. Die Ergebnisse sind in Tab.2 zusammengefaßt.

**Tab.2:**

| Schaumvermögen | | | | | | | |
|---|---|---|---|---|---|---|---|
| Bsp. | Tensid | Schaum (ml) nach x min | | | | | |
| | | 0,5 | 1 | 2 | 10 | 20 | 30 |
| 4 | Al | 1200 | 1700 | 2000 | | | |
| 5 | A2 | 1200 | 1700 | 2000 | | | |
| 6 | A3 | 1200 | 1700 | 2000 | | | |
| V2 | X | 1500 | 2000 | | | | |
| V3 | H1 | 540 | 600 | 650 | 800 | 1200 | 2000 |
| Legende: A1 = Alkylsulfat gemäß Herstellbeispiel 1 A2 = Alkylsulfat gemäß Herstellbeispiel 2 A3 = Alkylsulfat gemäß Herstellbeispiel 3 X = Dodecylbenzolsulfonat H1 = Alkylsulfat gemäß Vergleichsbeispiel V1 | | | | | | | |

### IV. Löslichkeitsuntersuchungen

**Rezepturen**. Die Löslichkeit der Alkylsulfate wurde in einer handelsüblichen Universalwaschmittel-Rezeptur untersucht:
20 Gew.-% Alkylsulfat gemäß Bsp.1 bis 4 bzw. V1
2 Gew.-% C_{12/14}-Fettalkohol mit 0,06 Gew.-% Polyethylenglycol (Molgewicht ca. 20.000)
5 Gew.-% Talgalkohol-40 EO-Addukt
35 Gew.-% Zeolith A
16 Gew.-% Natriumperborat
7 Gew.-% Wasserglas
15 Gew.-% Hilfsstoffe

Die Rezepturen wurden zunächst durch eine Schneckenpresse und anschließend durch eine Lochscheibe (Durchmesser der Löcher 1,1 mm) extrudiert und die resultierenden Stränge schließlich zu einem körnigen Granulat verarbeitet. Die Rezepturen I bis IV (mit den Alkylsulfaten gemäß den Herstellbeispielen 1 bis 4) sind erfindungsgemäß, die Rezeptur V (mit dem Alkylsulfat gemäß Herstellbeispiel V1) dient dem Vergleich.

**Testmethode**. 10 g der alkylsulfathaltigen Rezepturen I bis V wurden in jeweils 100 ml Wasser (30°C, 16°d) gelöst bzw. dispergiert. Nach 30, 120 und 300 s wurden die Lösungen bzw. Dispersionen abfiltriert, der Rückstand getrocknet und ausgewogen.

Die Ergebnisse der Löslichkeitsuntersuchungen in Tab.3 wiedergegeben:

**Tab.3:**

| Löslichkeitsversuche Prozentangaben in Gew.-% | | | | |
|---|---|---|---|---|
| Bsp. | Rezept. | Rückstand (%) nach | | |
| | | 30 s | 120 s | 360 s |
| 7 | I | 44,7 | 29,3 | 16,0 |
| 8 | II | 45,0 | 29,9 | 15,7 |
| 9 | III | 45,1 | 29,5 | 15,5 |
| 10 | IV | 44,3 | 28,9 | 15,2 |
| V4 | V | 75,4 | 61,5 | 45,7 |

## Patentansprüche

1. Verfahren zur Herstellung von Alkylsulfaten mit verbesserten Produkteigenschaften, bei dem man Fettalkohole ("A") der Zusammensetzung
0 bis 1 Gew.-% C₁₀
4 bis 23 Gew.-% C₁₂
1 bis 13 Gew.-% C₁₄
19 bis 52 Gew.-% C₁₆
34 bis 62 Gew.-% C₁₈
0 bis 3 Gew.-% C₂₀₊
in an sich bekannter Weise mit gasförmigem Schwefeltrioxid oder Chlorsulfonsäure sulfatiert und anschließend mit Basen neutralisiert.

2. Verfahren nach Anspruch 1, **dadurch gekennzeichnet**, daß man Gemische aus 70 bis 90 Gew.-% eines Fettalkohols ("B") der Zusammensetzung
0 bis 2 Gew.-% C₁₂
1 bis 7 Gew.-% C₁₄
25 bis 35 Gew.-% C₁₆
60 bis 67 Gew.-% C₁₈
0 bis 3 Gew.-% C₂₀
0 bis 1 Gew.-% C₂₂
und 10 bis 30, vorzugsweise 25 bis 30 Gew.-% eines Fettalkohols ("C") der Zusammensetzung
0 bis 3 Gew.-% C₁₀
48 bis 58 Gew.-% C₁₂
19 bis 24 Gew.-% C₁₄
9 bis 12 Gew.-% C₁₆
11 bis 15 Gew.-% C₁₈
0 bis 1 Gew.-% C₂₀
einsetzt.

3. Verfahren nach Anspruch 1, **dadurch gekennzeichnet**, daß man Gemische aus 70 bis 90 Gew.-% eines Fettalkohols ("D") der Zusammensetzung
0 bis 3 Gew.-% C₁₄
45 bis 55 Gew.-% C₁₆
45 bis 55 Gew.-% C₁₈
0 bis 3 Gew.-% C₂₀
und 10 bis 30 Gew.-% eines Fettalkohols ("C") der Zusammensetzung
0 bis 3 Gew.-% C₁₀
48 bis 58 Gew.-% C₁₂
19 bis 24 Gew.-% C₁₄
9 bis 12 Gew.-% C₁₆
11 bis 15 Gew.-% C₁₈
0 bis 1 Gew.-% C₂₀
einsetzt.

4. Verfahren nach Anspruch 1, **dadurch gekennzeichnet**, daß man Gemische aus 70 bis 95 Gew.-% eines Fettalkohols ("B") der Zusammensetzung
0 bis 2 Gew.-% C₁₂
1 bis 7 Gew.-% C₁₄
25 bis 35 Gew.-% C₁₆
60 bis 67 Gew.-% C₁₈
0 bis 3 Gew.-% C₂₀
0 bis 1 Gew.-% C₂₂
und 5 bis 30 Gew.-% eines Fettalkohols ("E") der Zusammensetzung
0 bis 2 Gew.-% C₁₀
65 bis 75 Gew.-% C₁₂
22 bis 30 Gew.-% C₁₄
0 bis 8 Gew.-% C₁₆
0 bis 1 Gew.-% C₁₈
einsetzt.

5. Verfahren nach Anspruch 1, **dadurch gekennzeichnet**, daß man Gemische aus 70 bis 95 Gew.-% eines Fettalkohols ("D") der Zusammensetzung
0 bis 3 Gew.-% C₁₄
45 bis 55 Gew.-% C₁₆
45 bis 55 Gew.-% C₁₈
0 bis 3 Gew.-% C₂₀
und 5 bis 30 Gew.-% eines Fettalkohols ("E") der Zusammensetzung
0 bis 2 Gew.-% C₁₀
65 bis 75 Gew.-% C₁₂
22 bis 30 Gew.-% C₁₄
0 bis 8 Gew.-% C₁₆
0 bis 1 Gew.-% C₁₈
einsetzt.

6. Verfahren nach den Ansprüchen 1 bis 5, **dadurch gekennzeichnet**, daß man als Produkt "B" ein Fettalkoholgemisch auf Basis tierischem Talg einsetzt.

7. Verfahren nach den Ansprüchen 1 bis 5, **dadurch gekennzeichnet**, daß man als Produkt "B" ein Fettalkoholgemisch auf Basis einer 1:1-Mischung aus Palmstearin und Rapsöl einsetzt.

8. Verfahren nach den Ansprüchen 1 bis 5, **dadurch gekennzeichnet**, daß man als Produkt "B" ein Fettalkoholgemisch auf Basis einer 1:1-Mischung aus Palmstearin und des C_{16/18}-Anteils aus Kokos- bzw. Palmkernöl einsetzt.

9. Verfahren nach den Ansprüchen 1 bis 8, **dadurch gekennzeichnet**, daß man als Produkte "C" und "E" Fettalkoholgemische auf Basis Kokosöl einsetzt.

10. Verfahren nach den Ansprüchen 1 bis 9, **dadurch gekennzeichnet**, daß man als Produkt "D" ein Fettalkoholgemisch auf Basis Palmstearin einsetzt.

11. Verfahren nach den Ansprüchen 1 bis 10, **dadurch gekennzeichnet**, daß man die Fettalkoholgemische zusammen mit 0,1 bis 10 Gew.-% - bezogen auf Fettalkohol - Polyethylenglycol (durchschnittliches Molekulargewicht 2000 bis 20.000) sulfatiert.

12. Verwendung von Alkylsulfaten nach dem Verfahren nach den Ansprüchen 1 bis 11 zur Herstellung von Waschmitteln.

13. Verwendung von Alkylsulfaten nach Anspruch 12, **dadurch gekennzeichnet**, daß man die Waschmittel durch Extrusionsverfahren herstellt.

14. Verwendung von Fettalkoholen ("A") der Zusammensetzung
0 bis 1 Gew.-% C₁₀
4 bis 23 Gew.-% C₁₂
1 bis 13 Gew.-% C₁₄
19 bis 52 Gew.-% C₁₆
34 bis 62 Gew.-% C₁₈
0 bis 3 Gew.-% C₂₀₊
zur Herstellung von Alkylsulfaten.

## Claims

1. A process for the production of alkyl sulfates having improved product properties, in which fatty alcohols ("A") having the following composition:
0 to 1% by weight C₁₀
4 to 23% by weight C₁₂
1 to 13% by weight C₁₄
19 to 52% by weight C₁₆
34 to 62% by weight C₁₈
0 to 3% by weight C₂₀₊
are sulfated in known manner with gaseous sulfur trioxide or chlorosulfonic acid and then neutralized with bases.

2. A process as claimed in claim 1, characterized in that mixtures of 70 to 90% by weight of a fatty alcohol ("B") having the following composition:
0 to 2% by weight C₁₂
1 to 7% by weight C₁₄
25 to 35% by weight C₁₆
60 to 67% by weight C₁₈
0 to 3% by weight C₂₀
0 to 1% by weight C₂₂
and 10 to 30 and preferably 25 to 30% by weight of a fatty alcohol ("C") having the following composition:
0 to 3% by weight C₁₀
48 to 58% by weight C₁₂
19 to 24% by weight C₁₄
9 to 12% by weight C₁₆
11 to 15% by weight C₁₈
0 to 1% by weight C₂₀
are used.

3. A process as claimed in claim 1, characterized in that mixture of 70 to 90% by weight of a fatty alcohol ("D") having the following composition:
0 to 3% by weight C₁₄
45 to 55% by weight C₁₆
45 to 55% by weight C₁₈
0 to 3% by weight C₂₀
and 10 to 30% by weight of a fatty alcohol ("C") having the following composition:
0 to 3% by weight C₁₀
48 to 58% by weight C₁₂
19 to 24% by weight C₁₄
9 to 12% by weight C₁₆
11 to 15% by weight C₁₈
0 to 1% by weight C₂₀
are used.

4. A process as claimed in claim 1, characterized in that mixtures of 70 to 95% by weight of a fatty alcohol ("B") having the following composition:
0 to 2% by weight C₁₂
1 to 7% by weight C₁₄
25 to 35% by weight C₁₆
60 to 67% by weight C₁₈
0 to 3% by weight C₂₀
0 to 1% by weight C₂₂
and 5 to 30% by weight of a fatty alcohol ("E") having the following composition:
0 to 2% by weight C₁₀
65 to 75% by weight C₁₂
22 to 30% by weight C₁₄
0 to 8% by weight C₁₆
0 to 1% by weight C₁₈.
are used.

5. A process as claimed in claim 1, characterized in that mixtures of 70 to 95% by weight of a fatty alcohol ("D") having the following composition:
0 to 3% by weight C₁₄
45 to 55% by weight C₁₆
45 to 55% by weight C₁₈
0 to 3% by weight C₂₀
and 5 to 30% by weight of a fatty alcohol ("E") having the following composition:
0 to 2% by weight C₁₀
65 to 75% by weight C₁₂
22 to 30% by weight C₁₄
0 to 8% by weight C₁₆
0 to 1% by weight C₁₈.
are used.

6. A process as claimed in claims 1 to 5, characterized in that a fatty alcohol mixture based on animal tallow is used as product "B".

7. A process as claimed in claims 1 to 5, characterized in that a fatty alcohol mixture based on a 1:1 mixture of palm stearin and rapeseed oil is used as product "B".

8. A process as claimed in claims 1 to 5, characterized in that a fatty alcohol mixture based on a 1:1 mixture of palm stearin and the C_{16/18} component of coconut oil or palm kernel oil is used as product "B".

9. A process as claimed in claims 1 to 8, characterized in that fatty alcohol mixtures based on coconut oil are used as products "C" and "E".

10. A process as claimed in claims 1 to 9, characterized in that a fatty alcohol mixture based on palm stearin is used as product "D".

11. A process as claimed in claims 1 to 10, characterized in that the fatty alcohol mixtures are sulfated together with 0.1 to 10% by weight - based on fatty alcohol - of polyethylene glycol (average molecular weight 2,000 to 20,000).

12. The use of the alkyl sulfates obtained by the process claimed in claims 1 to 11 for the production of detergents.

13. The use of alkyl sulfates as claimed in claim 12, characterized in that the detergents are produced by extrusion.

14. The use of fatty alcohols ("A") having the following composition:
0 to 1% by weight C₁₀
4 to 23% by weight C₁₂
1 to 13% by weight C₁₄
19 to 52% by weight C₁₆
34 to 62% by weight C₁₈
0 to 3% by weight C₂₀₊
for the production of alkyl sulfates.

## Revendications

1. Procédé de fabrication de sulfates d'alkyle ayant des propriétés de produit améliorées,
dans lequel on sulfate des alcools gras (A) de composition.
de 0 à 1 % en poids C₁₀
de 4 à 23 % en poids C₁₂
de 1 à 13 % en poids C₁₄
de 19 à 52 % en poids C₁₆
de 34 à 62 % en poids C₁₈
de 0 à 3 % en poids C₂₀₊
d'une manière connue en soi, avec de l'anhydride sulfurique gazeux ou avec de l'acide chlorosulfonique et ensuite on neutralise avec des bases.

2. Procédé selon la revendication 1,
caractérisé en ce qu'
on met en oeuvre des mélanges à base de 70 à 90 % en poids d'un alcool gras (B) de composition :
de 0 à 2 % en poids C₁₂
de 1 à 7 % en poids C₁₄
de 25 à 35 % en poids C₁₆
de 60 à 67 % en poids C₁₈
de 0 à 3 % en poids C₂₀
de 0 à 1 % en poids C₂₂
et de 10 à 30 % de préférence de 25 à 30 % en poids d'un alcool gras (C) de composition,
de 0 à 3 % en poids C₁₀
de 48 à 58 % en poids C₁₂
de 19 à 24 % en poids C₁₄
de 9 à 12 % en poids C₁₆
de 11 à 15 % en poids C₁₈
de 0 à 1 % en poids C₂₀

3. Procédé selon la revendication 1,
caractérisé en ce qu'
on met en oeuvre des mélanges à base de 70 à 90 % en poids d'un alcool gras (D) de composition :
de 0 à 3 % en poids C₁₄
de 45 à 55 % en poids C₁₆
de 45 à 55 % en poids C₁₈
de 0 à 3 % en poids C₂₀
et de 10 à 30 % en poids d'un alcool gras (C) de composition :
de 0 à 3 % en poids C₁₀
de 48 à 58 % en poids C₁₂
de 19 à 24 % en poids C₁₄
de 9 à 12 % en poids C₁₆
de 11 à 15 % en poids C₁₈
de 0 à 1 % en poids C₂₀

4. Procédé selon la revendication 1,
caractérisé en ce qu'
on met en oeuvre des mélanges à base de 70 à 95 % en poids d'un alcool gras (B) de composition :
de 0 à 2 % en poids C₁₂
de 1 à 7 % en poids C₁₄
de 25 à 35 % en poids C₁₆
de 60 à 67 % en poids C₁₈
de 0 à 3 % en poids C₂₀
de 0 à 1 % en poids C₂₂
et de 5 à 30 % en poids d'un alcool gras (E) de composition.
de 0 à 2 % en poids C₁₀
de 65 à 75 % en poids C₁₂
de 22 à 30 % en poids C₁₄
de 0 à 8 % en poids C₁₆
de 8 à 1 % en poids C₁₈

5. Procédé selon la revendication 1,
caractérisé en ce qu'
on met en oeuvre des mélanges à base de 70 à 95 % en poids d'un alcool gras (D) de composition :
de 0 à 3 % en poids C₁₄
de 45 à 55 % en poids C₁₆
de 45 à 55 % en poids C₁₈
de 0 à 3 % en poids C₂₀
et de 5 à 30 % en poids d'un alcool gras (« E ») de composition :
de 0 à 2 % en poids C₁₀
de 65 à 75 % en poids C₁₂
de 22 à 30 % en poids C₁₄
de 0 à 8 % en poids C₁₆
de 0 à 1 % en poids C₁₈

6. Procédé selon les revendications 1 à 5,
caractérisé en ce qu'
on met en oeuvre comme produit « B » un mélange d'alcools gras à base de « Suif » animal.

7. Procédé selon les revendications 1 à 5,
caractérisé en ce qu'
on met en oeuvre comme produit « B » un mélange d'alcools gras à base d'un mélange 1 : 1 de stéarine de palme et d'huile de colza.

8. Procédé selon les revendications 1 à 5,
caractérisé en ce qu'
on met en oeuvre comme produit « B » un mélange d'alcools gras à base d'un mélange 1 : 1 de stéarine de palme et de la fraction C₁₆/C₁₈ d'huile de coco ou d'huile de palmiste.

9. Procédé selon les revendications 1 à 8,
caractérisé en ce qu'
on met en oeuvre comme produits « C » et « E » des mélanges d'alcools gras à base d'huile de coco.

10. Procédé selon les revendications 1 à 9,
caractérisé en ce qu'
on met en oeuvre comme produit « D » un mélange d'alcools gras à base de stéarine de palme.

11. Procédé selon les revendications 1 à 10,
caractérisé en ce qu'
on sulfate les mélanges d'alcools gras, conjointement à de 0,1 à 10 % en poids, rapporté à l'alcool gras, de polyéthylèneglycol (poids moléculaire moyen 2000 à 20.000).

12. Utilisation de sulfates d'alkyle selon le procédé selon les revendications 1 à 11 pour la production de produits de lavage.

13. Utilisation de sulfates d'alkyle selon la revendication 12,
caractérisé en ce qu'
on fabrique les produits de lavage par un procédé d'extrusion.

14. Utilisation d'alcools gras (A) de composition :
de 0 à 1 % en poids C₁₀
de 4 à 23 % en poids C₁₂
de 1 à 13 % en poids C₁₄
de 19 à 52 % en poids C₁₆
de 34 à 62 % en poids C₁₈
de 0 à 3 % en poids C₂₀₊
en vue de la fabrication de sulfates d'alkyle.
